# EUROPEAN PATENT SPECIFICATION

(11) **EP 3 964 354 B1**
(45) Date of publication and mention of the grant of the patent: **14.08.2024**
(21) Application number: 21195341.9
(22) Date of filing: 07.09.2021
(51) Int. Cl.: B31B 50/10, B31B 50/16, B65B 55/08, B31B 50/00

(54) **FOOD WRAPPING STERILISING SYSTEM**
STERILISIERUNGSSYSTEM FÜR LEBENSMITTELVERPACKUNGEN
SYSTÈME DE STÉRILISATION D'EMBALLAGE ALIMENTAIRE

(30) Priority: 08.09.2020 IT 202000021169
(43) Date of publication of application: 09.03.2022
(73) Proprietor: Lacchinelli Paolo S.A.S. di Lacchinelli Ester Alessia & C., 26010 Bagnolo Cremasco Cremona (IT)
(72) Inventor: CROTTI, Paolo, 26013 Crema (CREMONA) (IT)
(74) Representative: Firmati, Leonardo

(56) References cited:
- EP-A1- 2 705 858
- WO-A1-2005/102842
- WO-A1-2019/026431
- DE-A1- 2 425 026
- US-A1- 2002 197 184

## Description

This invention relates to a system for conditioning wrappings for food products.

Due to the intrinsic nature of the food sector which is aimed at satisfying one of the primary needs of persons, the food sector is subject to checks and regulations aimed at guaranteeing the hygiene and safety of the products and of the persons themselves.

The recent situation, which has seen the spread of viruses at a global level, has lead to a greater attention, not only on the issues of hygiene and protection of human health, but also on the need to guarantee the wholesomeness of the entire production chain, especially in the food sector.

In this regard, it has been noted how the wrappings of food type can be, even by chance, carriers of microbial contamination, from the production, to the transport, up to the use for which the wrappings are designed.

This means that a wrapping contaminated by a microbial charge, or even worse by a viral charge, might be a carrier of diseases.

It has in fact been noted how the production of the wrappings in a controlled and sanitized environment, even though in line with food safety regulations, may not be sufficient to guarantee the integrity and the wholesomeness of the product and, consequently, the heath of the persons.

For example, contamination might occur during the process for producing the wrappings if an operator were carrying a viral and/or bacterial charge.

Similarly, a semi-finished product which were even just accidentally contaminated could adversely affect the wholesomeness of the food and the health of persons.

There are also prior art sterilizing systems, also applicable to paper, which, however, affect on the finished product, slowing down and complicating the process for making the wrapping. Moreover, these solutions have the drawback of not being able to guarantee that there is no contamination in the folds of the product made.

A system for conditioning food wrapping of known type, comprising an apparatus for sterilising paper product by means of UV radiation is disclosed in document US 2002/0197184. Moreover, when the wrapping is also made without contamination the same level of wholesomeness may not be similarly be guaranteed for the wrapping even during transport, placing at risk the efforts made to make the wrapping safe, if it is contaminated during a step after it has been made.

The aim of the invention is therefore to provide a system for conditioning wrappings for food products in such a way as to guarantee the wholesomeness of the wrapping, in terms of microbial charge, from the production up to its use.

A further aim of the invention is to provide a system for conditioning wrappings for food products which is effective in terms of reducing the microbial charge and efficient in terms of making the wrapping.

Another aim is to provide a system for conditioning wrappings for food products which is simple and inexpensive to make, compact and practical to use.

Yet another aim not forming part of the invention is to provide a method for making sterilized wrappings for food products which is versatile and adaptable to different types of wrappings. According to the invention, these aims and others are achieved by a system for conditioning wrappings for food products having the technical features described in the appended claims.

The technical features of the invention, with reference to the above-mentioned aims, are clearly described in the appended claims and its advantages are apparent from the detailed description which follows, with reference to the accompanying drawings which illustrate purely non-limiting example embodiments of the invention, in which:
- Figure 1 is a schematic side elevation view of an embodiment of the system for conditioning wrappings for food products according to the invention;
- Figure 2 is a schematic side elevation view of a detail of the system for conditioning wrappings for food products of Figure 1;
- Figure 3 schematically illustrates the steps of the method for making sterilized wrappings for food products according to the invention;
- Figure 4 schematically illustrates the steps of the method according to the previous drawing according to a different process configuration.

With reference to the accompanying drawings, the numeral 1 denotes in its entirety a system for conditioning wrappings I, made in accordance with the invention, hereinafter referred to simply with the numeral 1. According to the invention, the term semi-finished product S means a product, generally made in the form of reels BS, which requires further processing for it to be transformed into wrapping.

Moreover, according to the invention the term semi-finished paper product S means both a semi-finished product made solely from paper-based material and a semi-finished product made from different materials coupled to each other (for example, a plastic film) wherein at least one of the layers is made of paper, and semi-finished products with a paper-based matrix in which different elements or materials are dispersed. Sterilization means the processes designed to reduce the microbial charge of a surface.

The microbial charge is understood to mean viruses and/or bacteria.

The sanitization and the disinfection are considered as processes similar to sterilization.

However, the most general sanitizing and disinfection processes are not considered to be part of the above-mentioned definition, if they are aimed at cleaning a surface, that is to say, a mechanical operation for removing dirt or impurities from the surfaces.

Lastly, the term wrapping means both a product in the form of a sheet of flexible material designed for wrapping the food products and a bag with various shapes and designed for the same purpose.

The so-called MOCA, that is to say, materials and objects in contact with foodstuffs, are also considered as wrappings according to the invention, such as, for example, containers for transporting foodstuffs, provided they are paper-based, that is to say, formed or containing at least partly paper.

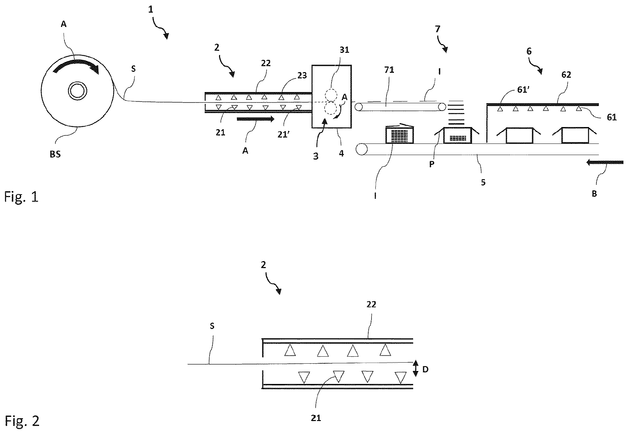

In particular, with reference to Figure 1, the system 1 is used for making wrappings for food products starting from a semi-finished paper product S. The system 1 comprises a first sterilization apparatus 2, movement means 3 and a cutting unit 4.

More specifically, the first apparatus 2 for sterilizing the semi-finished paper product S comprises a plurality of light sources 21 for irradiating the semi-finished product S with UV type radiation.

UV radiation is used to mean light radiation with a wavelength of between 10 and 400 nm.

Thanks to the UV radiation, the microbial charge present on the semi-finished product S is reduced between before and after the semi-finished product S.

With reference to Figure 1, the system 1 uses a plurality of light sources 21 and 21', each acting with UV type radiation.

In this way it is possible to increase the efficiency of the sterilization process.

That is to say, by providing a plurality of light sources 21 and 21' it is possible to continuously irradiate the semi-finished product S whilst this moves in a predetermined feed direction A.

By providing two or more light sources, it is in fact not necessary to block the operating flow to sterilize a particular point of the semi-finished product S, and then to sterilize the next point.

The same point of the semi-finished product S, sliding on the movement means 3, is exposed in succession to various light sources 21 and 21', thus achieving the same degree of sterilization and the same irradiation power in successive passages, rather than being stationary at a single point.

In this way, the entire conditioning system 1 is thus maintained in operation.

Advantageously, the plurality of light sources 21 and 21' is positioned in such a way that the distance from the semi-finished product S is constant.

According to embodiments not illustrated, the distance between the plurality of light sources 21 and 21' and the distance from the semi-finished element S is variable in order to perform different degrees of sterilization at different points of the semi-finished product S. Again with reference to Figure 1, the first sterilization apparatus 2 comprises at least two light sources 21 and 23 which act on opposite sides of the semi-finished product S.

In this way, the sterilization process is further improved, that is to say, it is possible to increase the speed of sliding of the movement means 3 and, consequently, of the entire system 1.

In fact, by positioning the light sources 21 and 23 in such a way as to irradiate both the lower surface and the upper surface of the semi-finished product S, it is possible to speed up the sterilization of the semi-finished product S, acting on both the surfaces exposed to the contamination.

Moreover, it has been noted how to strike the microorganisms several times, thus radiating them several times, makes the sterilization faster.

In this regard, in order to intensify the sterilization effect the light sources 21 and 23 face each other, in such a way that the radiation acts in the same surrounding of the semi-finished product S.

This solution is preferable, for example, if semi-finished products S are used which are particularly contaminated, or with greater thicknesses or even in the case of particularly porous semi-finished products S. According to other embodiments, the light sources 21 and 23 are offset from each other, in such a way that the radiation on the lower side and that on the upper side do not act simultaneously in the same surroundings of the semi-finished product, as illustrated in Figure 2. A similar effect can also be obtained by modulating the switching on and switching off of the set of light sources 21, 21' and 23.

This solution is preferable, for example, if semi-finished products S with reduced thicknesses are used or also in the case of semi-finished products S which are not particularly porous or for which the expected level of contamination is reduced.

Advantageously, at least one light source 21 and 23 is positioned at a distance D from the semi-finished element S of between 5 and 50 cm, as illustrated in Figure 2. Moving the light source towards the semi-finished product S prevents the flow of radiation from being diverted by obstacles or dust which can reduce the effect of the sterilization and adversely affect the integrity of the light source.

Moreover, it has been noted how the effectiveness of the sterilization depends on the distance D at which the light source is positioned.

This results in the need to identify the most effective distance D for the purposes of sterilizing a semi-finished paper product S.

Moreover, the distance D is such that the semi-finished product S remains at the correct temperature during the irradiation.

The light sources 21, 21' and 23 are positioned in a tunnel 22, as illustrated in Figure 1. The use of the tunnel 22 reduces the dispersion of radiation, improving the effectiveness of the sterilization process.

The movement means 3 feed the semi-finished product S to the light source 21, 21' and 23 at a speed of between 30 and 300 m/min in a predetermined feed direction A.

In this way it is possible to guarantee the continuous processing of the semi-finished product S.

The distance D is such that it is possible to move the semi-finished product at a speed of between 30 and 300 m/min, sterilising it at the same time.

In this way the entire conditioning system 1 is optimised.

With reference to Figure 1, the movement means 3 feed the semi-finished product S to the light source(s) by pulling the semi-finished product S.

The pulling applied to the semi-finished product S is calibrated in such a way as to move the semi-finished product S without applying stresses such as to adversely affect the integrity or cause plastic deformation. Suitable means for pulling the semi-finished product S are, for example, represented by cylinders 31 which flatten and rotate pulling the semi-finished product S, as illustrated in Figure 1.

According to embodiments not illustrated, the movement means 3 comprise conveyor belts to facilitate the movement of the semi-finished product S.

Moreover, the conveyor belts prevent the semi-finished product S from undergoing excessive stresses which could adversely affect the integrity of the semi-finished product during its forward movement.

For this purpose, according to some embodiments not illustrated, the conveyor belts contribute to the movement of the semi-finished product S.

This contribution occurs advantageously in the form of a thrust imparted to the movement of the semi-finished product S in the predetermined feed direction A.

The unit 4 for cutting the semi-finished product S is positioned downstream of the first sterilising apparatus 2 relative to the predetermined feed direction A.

That is to say, the process for sterilizing the semi-finished product S occurs only after the actual production of the wrapping I.

In effect, the cutting unit 4 cuts the semi-finished product S for making from it a plurality of wrappings I. The system 1 also comprises a feed line 5 for empty packaging P configured to feed a plurality of empty packaging P in a predetermined feed direction B, as illustrated in Figure 1.

The packaging P is designed to accommodate predetermined quantities of sterilized and cut wrappings I.

In this way it is possible to facilitate the transport of the wrappings I made by the system 1.

The feed line 5 comprises a second apparatus 6 for sterilization of empty packaging P.

More specifically, the second sterilization apparatus 6 comprises a plurality of light sources 61 for irradiating at least the inside surface of the packaging P with a UV type radiation.

The light sources 61 are positioned inside a tunnel 62, as illustrated in Figure 1.

In this way it is possible to avoid dispersion of radiation and a reduction of the effectiveness of the sterilization process.

Similarly to the first sterilization apparatus 2, the second sterilization apparatus 6 comprises a plurality of light sources 61 and 61' in such a way as to make more efficient and speed up the sterilization process.

Advantageously, the plurality of light sources 61 and 61' is positioned in such a way that the distance from the base from the packaging P is constant.

According to embodiments not illustrated, the distance between the plurality of light sources 61 and 61' and the distance from the base of the packaging P is variable in order to perform different degrees of sterilization at different points of the packaging P.

According to the invention, at least one light source 21, 21', 23, 61, 61' is in the form of a germicidal lamp. For example, germicidal lamps suitable for the purpose are the lamps which emit radiation in the UV-C spectrum, that is to say, between 100 and 280 nm.

Lamps designed for the purpose are, for example, mercury light bulbs, UV-C light bulbs, LED UV-C lamps, lamps in UVGI spectrum ("Ultraviolet germicidal irradiation"), lamps which use microwave induction.

The system 1 also comprises a packaging unit 7 configured to place the plurality of wrappings I inside the packaging P.

With reference to Figure 1, the packaging unit 7 comprises conveyor belts 71, as illustrated in Figure 1 to facilitate the transport and the accumulation of wrappings I at the outfeed from the cutting unit 4.

The packaging unit 7 is located downstream of the first sterilization apparatus 2 in the predetermined feed direction A and downstream of the second sterilization apparatus 6 in the predetermined feed direction B. With reference to Figure 1, the predetermined feed directions A and B are opposite to each other. According to embodiments not illustrated, the predetermined feed directions A and B are in the same direction.

In that way it is possible to optimise the spaces, being able to reduce the longitudinal extension of the system 1.

In any case, even in this configuration, the packaging unit 7 is located downstream of the first sterilization apparatus 2 and of the second sterilization apparatus 6, so that both the wrapping I and the packaging P are precisely sterilized.

The system 1 comprises a command and control unit configured to power and control the first and second sterilization apparatus (2, 6).

According to the invention, the command and control unit is configured for setting the process parameters such as the switching on and switching off time of the light sources 21, 21', 23, 61 and 61'.

The command and control unit is also configured to control the movement means 3 and the feed line 5.

According to the invention, the command and control unit is configured for setting the process parameters such as the feed speed according to the predetermined feed directions A and B. Moreover, the command and control unit is configured for setting the cutting unit 4.

According to the invention, the command and control unit is configured for setting the process parameters such as the size and shape of the wrapping I.

The command and control unit is also configured to adjust and check the packaging of the wrappings I.

According to the invention, the command and control unit is configured for setting the process parameters such as the time the packaging P remains in the packaging unit 7.

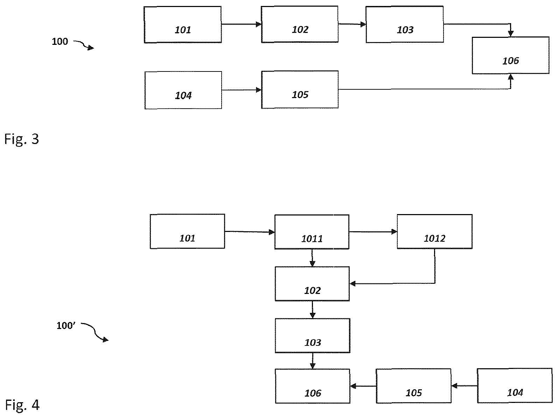

In use, the semi-finished product S starting from the housing section of the reel BS advances towards the first sterilization apparatus 2 and the cutting unit 4 in order to make sterilized wrappings I for food products, as illustrated in steps in the process 100 of Figure 3. More specifically, after preparing the semi-finished paper product S, step 101, this is moved at a speed of between 30 and 300 m/min through the first sterilization apparatus 2.

At this point, the semi-finished product S is sterilized with UV radiation to reduce the microbial charge of said semi-finished product S between before and after the first sterilization apparatus 2, step 102. Advantageously, the radiation used is of the UV-C type. After being sterilized, the semi-finished product S is cut to make from it a plurality of wrappings I, step 103.

At the same time, if the process is to be made more efficient, or at a different moment for different requirements, the packaging P is sterilized.

More specifically, the packaging P, not illustrated, moves forward towards the second sterilization apparatus 6 in order to package the sterilized packaging P from a section for housing the packaging P.

After preparing an empty packaging P for receiving predetermined quantities of sterilized and cut wrappings I, the step 104, the packaging P is moved by the feed line 5 of empty packagings for passing through the second sterilization apparatus 6.

After that, at least the inside surface of the packaging P is sterilized with UV radiation, step 105.

Subsequently, the predetermined quantity of wrappings I is inserted into the sterilized pack P, step 106.

More specifically, according to the invention, the duration of the sterilization step 102 of the semi-finished product S is in the order of seconds, advantageously less than one minute, and preferably between 3 and 15 seconds.

These values are modulated on the basis of the feed speed of the semi-finished product S.

Figure 4 illustrates the process 100' for making sterilized wrappings I for food products wherein the step 101 of preparing the semi-finished paper product S also comprises the step of selecting at least one plastic film coupled at least partly to at least one surface of the semi-finished product S, step 1012.

The plastic film, coupled to the semi-finished product S, increases the tensile and shear strength of the semi-finished paper product S.

The semi-finished product S coupled with the plastic film is then subjected to steps 102 to 106 as described above.

According to other embodiments, the step 101 of preparing the semi-finished paper product S also comprises a step of moulding the semi-finished product, referred to as step 1011.

As illustrated in Figure 4, the step 1011 of moulding the semi-finished product S is optionally associated with a further step of selecting a plastic film coupled at least partly to at least one surface of the semi-finished product S, step 1012.

According to embodiments not illustrated, the step 1011 of moulding the semi-finished product S occurs after the step 1012 of selecting a plastic film.

The moulding step 1011 is performed before or after the step of selecting the plastic film depending on the type of semi-finished paper product S and its mechanical characteristics, in such a way as not to modify in a negative manner the final features of the semi-finished product S.

If the method described above uses of a moulded semi-finished product S, that is to say, which has been subjected to the moulding step 1011, the sterilization of the moulded semi-finished product S occurs by acting on the side of the semi-finished product S which is moulded, to guarantee a correct sterilization of the semi-finished product S, and therefore of the inks used. According to other embodiments, the sterilization of the moulded semi-finished product S occurs by acting on the side of the semi-finished product S which is not printed, so as not to risk adversely affecting the printed side. According to these embodiments, care will be taken to guarantee the sterilization of the inks before starting the process for processing the semi-finished product S. For this purpose, the parameters such as the speed of the movement means 3, the number of light sources activated and the distance D of the light sources from the semi-finished product S are suitably modulated to guarantee the effectiveness of the process for making sterilized wrappings for food products.

Thanks to the method 100 and 100' described above, a sterilized wrapping I for food products is advantageously made.

The system 1 for conditioning wrappings I for food products and the method 100 and 100' for making sterilized wrappings I for food products according to the invention achieve the preset aims and brings important advantages.

A first advantage of the conditioning system 1 and of the method 100 and 100' according to the invention is the possibility of making wrappings for sterilized food products such as to eliminate or reduce the microbial, viral and/or bacterial charge of the wrapping to non-harmful values for human health, so as to make the production process healthy.

With the system 1 and the method 100 and 100' according to the invention it is in fact possible to reduce the risk of contamination during the process for making the wrapping I and packaging it.

Another advantage is due to the fact that the integrity of the wrappings I at the outfeed from the system 1 and during their transport is guaranteed.

This solution has the advantage of making the wrapping I integral in terms of sterilization even during transport, thanks to the sterilization of the packaging P.

This guarantees the wholeness of the wrapping I, from production to its direct use.

Another advantage is the possibility of sterilizing the semi-finished product S during the work flow, preventing the production from being interrupted or slowed down.

Another advantage is due to the fact that the system 1 and the method 100 and 100' for making sterilized wrappings I according to the invention allow the microbial charge to be reduced between before and after processing the semi-finished product S.

That is to say, between the infeed of the semi-finished product inside the first sterilization apparatus 2 and the wrapping of the semi-finished product S.

Yet another advantage is due to the fact that, with the system 1 and the method 100 and 100' for making sterilized wrappings I, the sterilization process occurs before the actual wrapping is made, thus improving the efficiency of the process, being able to prevent the sterilization of each individual wrapping, that is to say, after being made.

A further advantage is due to the fact that the system 1 and the method 100 and 100' for making wrappings according to the invention can be adapted to different types of semi-finished paper products, both coupled to a plastic film and moulded, modulating the speed of the system 1, the intensity of the light source(s) and the distance D of the light source(s) from the semi-finished product S.

## Claims

1. A food wrapping conditioning system (I), starting from a semi-finished paper product (S), comprising:
- a first apparatus (2) for sterilising said semi-finished paper product (S) comprising a plurality of light sources (21, 21', 23) designed to irradiate said semi-finished product (S) with UV radiation, said light sources (21, 21', 23) being positioned in a tunnel (22);
- movement means (3) designed to feed said semi-finished product (S) at said plurality of light sources (21, 21', 23) at a speed of between 30 and 300 m/min in a predetermined feed direction (A);
- a unit (4) for cutting said semi-finished product (S) positioned downstream of said first sterilising apparatus (2) relative to said predetermined feed direction (A) and designed to cut said semi-finished product (S) for making from it a plurality of said wrappings (I), **characterized in that** it comprises a feed line (5) for empty packaging (P) configured to feed a plurality of empty packaging (P) in a predetermined feed direction (B), said packaging (P) being designed to receive predetermined quantities of said sterilized and cut wrappings (I), said feed line (5) comprising a second apparatus (6) for sterilising said empty packaging (P), said second sterilization apparatus (6) comprising a plurality of light sources (61, 61') designed to irradiate at least the inner surface of said packaging (P) with a UV type radiation, said light sources (61, 61') being positioned inside a tunnel (62), and **in that** it comprises:
- a packaging unit (7) configured to place the plurality of wrappings (I) in said packaging (P), said packaging unit (7) being located downstream of said first sterilization apparatus (2) in said predetermined feed direction (A) and downstream of said second sterilization apparatus (6) in said predetermined feed direction (B),
- a command and control unit configured to:
- feed and control said first and second sterilization apparatus (2, 6), said command and control unit being configured for setting the process parameters such as the switching on and switching off time of said light sources (21, 21', 23, 61, 61'),
- control said movement means (3) and said feed line (5), said command and control unit being configured for setting the process parameters such as the feed speed according to said predetermined feed directions (A, B),
- set said cutting unit (4), said command and control unit being configured for setting the process parameters such as the size and shape of said wrapping (I),
- adjust and control the packaging of said wrappings (I), said command and control unit being configured for setting the process parameters such as the time said packaging (P) remains in said packaging unit (7).

2. The system according to claim 1, wherein said at least one light source (21, 21', 23) is placed at a distance (D) from said semi-finished product (S) of between 5 and 50 cm.

3. The system according to claim 1, wherein said first sterilization apparatus (2) comprises at least two light sources (21, 21', 23) designed to act on opposite sides of said semi-finished product (S).

4. The system according to claim 1, wherein said at least one light source (21, 21', 23, 61, 61') is in the form of a germicidal lamp which is able to emit UV-C type radiation.

## Patentansprüche

1. Aufbereitungssystem für Lebensmittelverpackungen (I), ausgehend von einem halbfertigen Papierprodukt (S), umfassend:
- eine erste Vorrichtung (2) zum Sterilisieren des halbfertigen Papierprodukts (S), umfassend eine Vielzahl von Lichtquellen (21, 21', 23), die ausgestaltet sind, um das halbfertige Produkt (S) mit UV-Strahlung zu bestrahlen, wobei die Lichtquellen (21, 21', 23) in einem Tunnel (22) positioniert sind;
- Bewegungsmittel (3), die ausgestaltet sind, um das halbfertige Produkt (S) den Lichtquellen (21, 21', 23) bei einer Geschwindigkeit zwischen 30 und 300 m/min in einer vorbestimmten Zuführungsrichtung (A) zuzuführen;
- eine Einheit (4) zum Schneiden des halbfertigen Produkts (S), die stromabwärts der ersten Sterilisierungsvorrichtung (2) relativ zur vorbestimmten Zuführungsrichtung (A) positioniert und ausgestaltet ist, um das halbfertige Produkt (S) zu schneiden, um daraus eine Vielzahl von Verpackungen (I) herzustellen, **dadurch gekennzeichnet, dass** es eine Zuführungsanlage (5) für leere Umverpackungen (P) umfasst, die ausgelegt ist, um eine Vielzahl von leeren Umverpackungen (P) in einer vorbestimmten Zuführungsrichtung (B) zuzuführen, wobei die Umverpackungen (P) ausgestaltet sind, um vorbestimmte Mengen von sterilisierten und geschnittenen Verpackungen (I) zu erhalten, wobei die Zuführungsanlage (5) eine zweite Vorrichtung (6) zum Sterilisieren der leeren Umverpackungen (P) umfasst, wobei die zweite Sterilisierungsvorrichtung (6) eine Vielzahl von Lichtquellen (61, 61') umfasst, die ausgestaltet sind, um mindestens die innere Oberfläche der Umverpackung (P) mit einer Strahlung vom UV-Typ zu bestrahlen, wobei die Lichtquellen (61, 61') im Tunnel (62) positioniert sind, und dadurch, dass es Folgendes umfasst:
- eine Umverpackungseinheit (7), die ausgelegt ist, um die Vielzahl von Verpackungen (I) in der Umverpackung (P) zu platzieren, wobei die Umverpackungseinheit (7) stromabwärts der ersten Sterilisierungsvorrichtung (2) in der vorbestimmten Zuführungsrichtung (A) und stromabwärts der zweiten Sterilisierungsvorrichtung (6) in der vorbestimmten Zuführungsrichtung (B) befindlich ist;
- eine Befehls- und Steuereinheit, die ausgelegt ist, um
- die erste und die zweite Sterilisierungsvorrichtung (2, 6) zu beschicken und zu steuern, wobei die Befehls- und Steuereinheit ausgelegt ist, um die Prozessparameter wie die Ein- und Ausschaltzeit der Lichtquellen (21, 21', 23, 61, 61') festzulegen;
- die Bewegungsmittel (3) und die Zuführungsanlage (5) zu steuern, wobei die Befehls- und Steuereinheit ausgelegt ist, um die Prozessparameter wie die Zuführgeschwindigkeit nach den vorbestimmten Zuführungsrichtungen (A, B) festzulegen;
- die Schneideinheit (4) einzustellen, wobei die Befehls- und Steuereinheit ausgelegt ist, um die Prozessparameter wie die Größe und Form der Verpackung (I) festzulegen;
- die Umverpackung der Verpackungen (I) zu regeln und zu steuern, wobei die Befehls- und Steuereinheit ausgelegt ist, um die Prozessparameter wie die Zeit, in der die Umverpackung (P) in der Umverpackungseinheit (7) bleibt, festzulegen.

2. System nach Anspruch 1, wobei die mindestens eine Lichtquelle (21, 21', 23) in einer Entfernung (D) vom halbfertigen Produkt (S) zwischen 5 und 50 cm platziert ist.

3. System nach Anspruch 1, wobei die erste Sterilisierungsvorrichtung (2) mindestens zwei Lichtquellen (21, 21', 23) umfasst, die ausgestaltet sind, um auf entgegengesetzte Seiten des halbfertigen Produkts (S) einzuwirken.

4. System nach Anspruch 1, wobei die mindestens eine Lichtquelle (21, 21', 23, 61, 61') in der Form einer Entkeimungslampe ausgebildet ist, die in der Lage ist, eine Strahlung vom UV-C-Typ auszusenden.

## Revendications

1. Système de conditionnement d'emballages alimentaires (I), à partir d'un produit semi-fini en papier (S), comprenant :
- un premier appareil (2) servant à stériliser ledit produit semi-fini en papier (S), comprenant une pluralité de sources lumineuses (21, 21', 23) conçues pour irradier ledit produit semi-fini (S) avec un rayonnement UV, lesdites sources lumineuses (21, 21', 23) étant positionnées dans un tunnel (22) ;
- des moyens de déplacement (3) conçus pour alimenter ledit produit semi-fini (S) à ladite pluralité de sources lumineuses (21, 21', 23) à une vitesse comprise entre 30 et 300 m/min dans une direction d'alimentation (A) prédéterminée ;
- une unité (4) servant à découper ledit produit semi-fini (S), positionnée en aval dudit premier appareil de stérilisation (2) par rapport à ladite direction d'alimentation (A) prédéterminée et conçue pour découper ledit produit semi-fini (S) pour en faire une pluralité desdits emballages (I), **caractérisé en ce qu'**il comprend une ligne d'alimentation (5) pour empaquetages vides (P) configurée pour alimenter une pluralité d'empaquetages vides (P) dans une direction d'alimentation (B) prédéterminée, ledit empaquetage (P) étant conçu pour recevoir des quantités prédéterminées desdits emballages (I) stérilisés et découpés, ladite ligne d'alimentation (5) comprenant un deuxième appareil (6) pour stériliser ledit empaquetage vide (P), ledit deuxième appareil de stérilisation (6) comprenant une pluralité de sources lumineuses (61, 61') conçues pour irradier au moins la surface intérieure dudit empaquetage (P) avec un rayonnement de type UV, lesdites sources lumineuses (61, 61') étant positionnées à l'intérieur d'un tunnel (62), et **en ce qu'**il comprend :
- une unité d'empaquetage (7) configurée pour placer la pluralité d'emballages (I) dans ledit empaquetage (P), ladite unité d'empaquetage (7) étant située en aval dudit premier appareil de stérilisation (2) dans ladite direction d'alimentation (A) prédéterminée et en aval dudit deuxième appareil de stérilisation (6) dans ladite direction d'alimentation (B) prédéterminée,
- une unité de commande et de contrôle configurée pour :
- alimenter et contrôler lesdits premier et deuxième appareils de stérilisation (2, 6), ladite unité de commande et de contrôle étant configurée pour régler les paramètres du processus tels que les heures d'allumage et d'extinction desdites sources lumineuses (21, 21', 23, 61, 61'),
- contrôler lesdits moyens de déplacement (3) et ladite ligne d'alimentation (5), ladite unité de commande et de contrôle étant configurée pour régler les paramètres du processus tels que la vitesse d'alimentation selon les directions d'alimentation (A, B) prédéterminées,
- régler ladite unité de coupe (4), ladite unité de commande et de contrôle étant configurée pour régler les paramètres du processus tels que la taille et la forme dudit emballage (I),
- ajuster et contrôler l'empaquetage desdits emballages (I), ladite unité de commande et de contrôle étant configurée pour régler les paramètres du processus tels que le temps pendant lequel ledit empaquetage (P) reste dans ladite unité d'empaquetage (7).

2. Système selon la revendication 1, dans lequel ladite au moins une source lumineuse (21, 21', 23) est placée à une distance (D) dudit produit semi-fini (S) comprise entre 5 et 50 cm.

3. Système selon la revendication 1, dans lequel ledit premier appareil de stérilisation (2) comprend au moins deux sources lumineuses (21, 21', 23) conçues pour agir sur des côtés opposés dudit produit semi-fini (S).

4. Système selon la revendication 1, dans lequel ladite au moins une source lumineuse (21, 21', 23, 61, 61') se présente sous la forme d'une lampe germicide capable d'émettre un rayonnement de type UV-C.
